# EUROPEAN PATENT SPECIFICATION

(11) **EP 1 267 716 B1**
(45) Date of publication and mention of the grant of the patent: **11.08.2010**
(21) Application number: 01922143.1
(22) Date of filing: 30.03.2001
(51) Int. Cl.: A61B 5/055, A61K 49/06

(54) **METHOD OF MAGNETIC RESONANCE IMAGING**
VERFAHREN ZUR MAGNETRESONANZBILDGEBUNG
PROCEDE D'IMAGERIE PAR RESONANCE MAGNETIQUE

(30) Priority: 31.03.2000 GB 0007872
(43) Date of publication of application: 02.01.2003
(73) Proprietor: GE HEALTHCARE AS, 0401 Oslo (NO)
(72) Inventor: JOHANSSON, Lars, SE-741 96 Knivsta (SE)
(74) Representative: Wulff, Marianne Weiby
(86) International application number: PCT/NO2001/000137
(87) International publication number: WO 2001/074245

(56) References cited:
- WO-A-00/72037
- WO-A-96/27394
- WO-A-99/06849
- US-A- 5 855 868
- SMALL W C ET AL: "Dual Contrast Enhancement of Both T1- and T2-Weighted Sequences Using Ultrasmall Superparamagnetic Iron Oxide" MAGNETIC RESONANCE IMAGING, vol. 11, no. 5, 1993, pages 645-654, XP002901813
- J. R. PANTING ET AL.: "First-Pass Myocardial Perfusion Imaging and Equilibrium Signal Changes Using the Intravascular Contrast Agent NC100150 Injection" JOURNAL OF MAGNETIC RESONANCE IMAGING, vol. 10, 1999, pages 404-410,

## Description

This invention relates to magnetic resonance (MR) imaging of blood perfusion in vascularized organs of interest, e.g. the myocardium, the kidneys, the brain, the liver and in particular imaging of myocardial perfusion.

Viewed from one aspect the invention provides a method of first pass, contrast enhanced organ perfusion magnetic resonance imaging of a human or vascularized animal subject, wherein a MR contrast agent composition is administered into the vasculature of said subject and magnetic resonance images are generated of slices through a vascularized organ of said subject during the passage of said agent through the vasculature of said organ, and wherein said contrast agent comprises a particulate superparamagnetic metal oxide and in that said images are generated using pre-saturation slabs and a T₂-weighted Turbo (fast) spin-echo sequence and wherein said vascularized organ preferably is the myocardium, the kidneys, the brain or the liver, and more preferably the myocardium.

The article by W. C. Small et al., "Dual contrast enhancement of both T1- and T2-weighted sequences using ultrasmall superparamagnetic iron oxide", Magnetic Resonance Imaging, vol. 11, 1993, p. 645-954, describes the use of BMS 180549, an ultrasmall superparamagnetic iron particulate agent, as a contrast agent on T₁- and T₂-weighted Turbo spin-echo sequences.

In the field of cardiac imaging using magnetic resonance there has recently been great interest in the study of myocardial perfusion. Perfusion deficits revealed by such studies indicate areas of actual or potential damage to heart muscle, for example as a result of coronary infarction or total or partial arterial occlusion, and the technique can be used to monitor and assess the success of pharmacological or surgical attempts to reperfuse areas of perfusion deficit.

To date the major thrust has been in the development of first-pass myocardial perfusion imaging using T₁-weighted sequences and low molecular weight gadolinium chelate MR contrast agents, such as gadodiamide (Omniscan^{®}, Nycomed Amersham) and gadopentetate (Magnevist^{®}, Schering), agents which distribute throughout the extracellular space, i.e. within both the blood vessels and the interstitium. Generally the MR image acquisition sequences used have been very fast gradient-echo sequences or multishot echo planar imaging (EPI) sequences which rely on fast read out and a short TR in order to achieve sufficient resolution and acquire images of multiple slices through the heart in each heart beat. Such sequences usually acquire 3 or 4 slices each heart beat or 6 to 8 interleaved slices in two heart beats and the in-plane resolution is generally about 2 to 3 mm.

We have now found that increased resolution can be achieved by using a particulate superparamagnetic metal oxide contrast agent, in particular an ultrasmall superparamagnetic iron oxide (USPIO) contrast agent, rather than a soluble gadolinium chelate, and by the use of a T₂-weighted Turbo (fast) spin-echo sequence.

The invention therefore provides a method of first pass, contrast enhanced myocardial perfusion magnetic resonance imaging of a human or vascularized animal (e.g. mammalian, avian or reptilian, but preferably mammalian) subject, wherein an MR contrast agent composition is administered into the vasculature of said subject and magnetic resonance images are generated of slices through the heart of said subject during the passage of said agent through the vasculature of said heart, and wherein said contrast agent comprises a particulate superparamagnetic metal oxide (preferably an USPIO) and in that said images are generated using pre-saturation slabs and a T₂-weighted Turbo (fast) spin-echo sequence.

Viewed from a further aspect the invention provides a method of generating MR images of a human or vascularized animal subject, previously administered with a MR contrast agent composition comprising a particulate superparamagnetic metal oxide (preferably an USPIO), which method comprises generating a first pass contrast enhanced organ perfusion MR image of slices through the vascularized organ of said subject and in that said images are generated using pre-saturation slabs and a T₂-weighted Turbo (fast) spin echo sequence. Preferably the organ is the myocardium, the kidneys, the brain or the liver, and more preferably the myocardium,

Viewed from a still further aspect the invention provides the use of a particulate superparamagnetic metal oxide (preferably an USPI0) for the manufacture of a MR contrast agent composition for use in a method of diagnosis involving first pass contrast enhanced organ perfusion magnetic resonance imaging of perfusion deficits in a vascularized organ using pre-saturation slabs and a T₂-weighted Turbo (fast) spin-echo sequence.

Preferably the organ is the myocardium (heart), the kidneys, the brain or the liver and more preferably the organ is the myocardium (heart).

The methods of the invention are preferably used to generate images of at least two slices per vascularized organ cycle, preferably cardiac cycle, e.g. 2 to 10 slices/cycle, preferably 3 to 5 slices/cycle. Where the image of the heart is to be built up from images from more than one cardiac cycle, e.g. from two or less preferably three cycles, the slices from separate cycles are preferably interleaved.

Unlike the gadolinium chelates whose contrast effect in the vasculature is generally due to their T₁ reducing effect, superparamagnetic contrast agents, especially USPIOs, have both T₁ and T₂/T₂* reducing effects, the latter of which can dominate at high concentrations. Thus, since on first pass of a superparamagnetic contrast agent through the heart's vasculature the contrast agent concentration can be relatively high, the T₂/T₂* effect can be used for the first pass imaging in a T₂-weighted Turbo (fast) spin echo sequence. Such a sequence is preferably used in a single shot acquisition (i.e. where an entire image rather than a single image line is read out per rf-excitation) allowing the use of pre-saturating slabs, generally in the phase-encoding direction. In this way outer volume suppression, i.e. of the extra-cardiac signal, can be achieved without unduly prolonging the image acquisition time since a smaller field of view (FOV), and hence fewer phase-encoding lines, are necessary to achieve the required image resolution. Thus the field of view for the cardiac image can be reduced significantly, e.g. to a value commensurate with the heart cross section dimension, so causing an increase in resolution. This reduction in FOV cannot be done using conventional T₁-weighted gradient echo imaging with gadolinium chelates since the pre-saturation pulses would need to be applied within each sequence cycle, i.e. within each TR, and would accordingly increase TR so much that it would not be possible to acquire sufficient slices within each cardiac cycle.

The superparamagnetic MR contrast agent used according to the invention may be any physiologically tolerable agent comprising superparamagnetic iron oxide (or doped iron oxide) particles. However particles which have a blood half life (measured for example in the pig) of at least 10 minutes, preferably at least 30 minutes, more preferably at least 1 hour, are especially preferred. Such particles are often referred to as blood pool contrast agents. Generally the contrast agent will be a particulate material having a particle size of 1 to 8000 nm, preferably 5 to 500 nm, more preferably 5 to 100nm. Blood residence times for such particles can be enhanced by provision of an opsonization inhibiting coating, e.g. polyalkylene oxides (e.g. PEG), glycosaminoglycans (e.g. heparin or heparinoids, dermatan, hyaluronic acid, keratan, chondroitin, etc.). Particularly suitable as USPIO agents are dextran or carboxy-dextran-coated USPIOs, the degraded starch coated USPIOs of WO97/2507 (preferably also provided with a PEG coating), AMI 7228 and the particulate agents described in WO95/05669, WO91/12526, WO91/12025, WO90/01899, WO88/00060, WO92/11037 and WO90/01295.

The superparamagnetic agents are especially preferably members of the subclass known as ultra small superparamagnetic iron oxides (USPIO). More particularly the superparamagnetic agent is preferably a water-dispersible material comprising magnetic iron oxide particles having on their surfaces (e.g. as a coating), an optionally modified carbohydrate or polysaccharide or derivative thereof, e.g. a glucose unit containing optionally modified polysaccharide or derivative thereof, preferably an optionally modified dextran or starch or derivative thereof, for example a cleaved (e.g. oxidatively cleaved) starch or carboxylated dextran. Such iron oxide complexes preferably also comprise a further material (e.g. coating material), especially one which inhibits opsonization, e.g. a hydrophilic polymer, preferably a functionalized polyalkylene oxide, more preferably a functionalized polyethylene glycol (PEG), in particular methoxy PEG phosphate (MPP).

The iron oxide complexes preferably have a core (i.e. iron oxide particle) diameter (mode diameter) of 1 to 15 nm, more preferably 2-10 nm, especially 3-7 nm, a total diameter (mode particle size) of 1 to 100 nm, more preferably 5-50 nm, especially preferably 10-25 nm, an r₂/r₁ ratio at 0.47T and 40°C of less than 3, more preferably less than 2.3, still more preferably less than 2.0, especially preferably less than 1.8. The saturation magentization (Msat) at 1T is preferably 10 to 100 emu/gFe, more preferably 30-90 emu/gFe.

One such agent currently undergoing clinical trials is known as NC100150 a blood pool contrast agent coated with a starch residue and further coated with a PEG component, (Clariscan^{™}, Nycomed Imaging AS, Oslo, Norway), e.g. disclosed in WO 97/25073 (see Ex. 12).

In the methods of the invention, the superparamagnetic contrast agent composition is preferably administered by bolus injection into the vasculature, particularly a vein, e.g. a limb vein or in the case of an animal a tail vein. The bolus is preferably administered over a period of less than 3 minutes, more preferably less than 100 seconds, still more preferably less than 60 seconds, e.g. 0.3 to 10 seconds. Contrast medium injection rates will desirably be in the range 0.01 to 10 mL/sec, especially 0.3 to 3.0 mL/sec. The bolus should desirably be as tight as possible and may be sharpened by the use of a physiological saline chaser.

In the methods of the invention, the superparamagnetic MR contrast agent is preferably administered in a dose of 0.5 to 8 mg Fe/kg bodyweight, more preferably 1 to 6 mg Fe/kg, especially 2 to 5 mg Fe/kg.

The superparamagnetic contrast agent may be formulated with conventional pharmaceutical carriers and excipients. Typically they will be in aqueous dispersion form, e.g. at an iron content of 10 to 50 mg Fe/mL, preferably 20 to 40 mg Fe/mL. Excipients that may be present include pH modifiers, chelating agents, viscosity modifiers, osmolality modifiers, etc.

The T₂-weighted Turbo (fast) spin echo sequence is preferably effected with a repetition time of one cardiac cycle and a FOV which is 80 to 120% of the cardiac dimension, e.g. 20 to 150 mm, especially 50 to 100 mm. Turbo (fast) spin echo imaging is described in Henning et al in MRM 3: 823-833 (1986). Pre-saturation slabs are positioned so as to avoid signal from phase wrapping and so as to enable the small FOV values to be achieved.

The invention will now be described further with reference to the following non-limiting Example and the accompanying drawing in which:

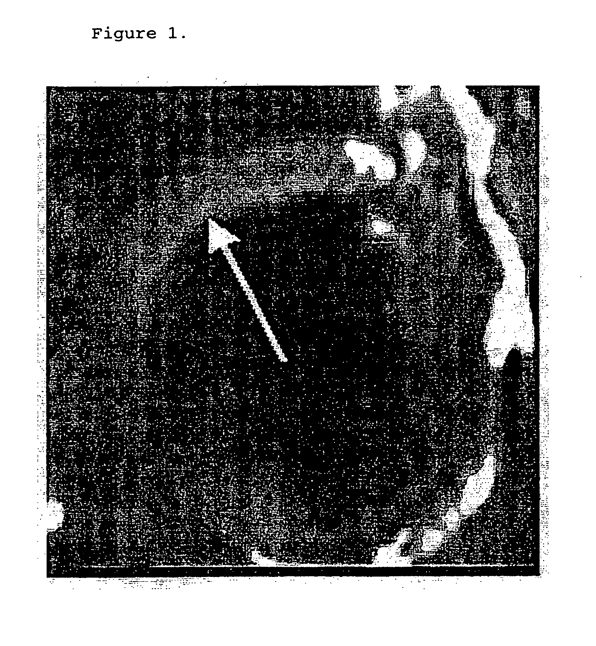

Figure 1 is a short-axis view of a pig heart with an occluded left anterior descending coronary artery generated by first pass myocardial imaging using a T₂-weighted single shot Turbo (fast) spin echo sequence following iv administration of an USPIO content agent.

An aqueous suspension of an USPIO blood pool MR contrast agent prepared according to the description in Example 12 of WO97/2507 was used in this Example. The characteristics of this suspension were: [Fe] = 30.2 mg Fe/mL; density 1.0589 g/mL; r₁ = 19.3 s⁻¹mM⁻¹; r₂ = 31.2 s⁻¹mM⁻¹; r₂/r₁ = 1.61 (at 20 MHz and 37°C) ; saturation magnetization (Msat) = 84 emu/g Fe.

### Example 1

### In vivo imaging

5 mg Fe/kg bodyweight of the contrast agent composition was administered by bolus injection over 2 seconds into the ear vein of a pig (30 kg) in which the left anterior descending coronary artery had been surgically ligated.

During first pass of the contrast agent (approximately 10 seconds after administration) images of the pig's heart were generated using a 1.5T Philips magnetic resonance imaging apparatus operating a T₂-weighted single shot Turbo (fast) spin echo sequence with TE at 200 ms and a FOV limited to 80 mm using pre-saturation slabs applied in the phase encoding direction outside the FOV. One slice image obtained in this way is shown in Figure 1 of the accompanying drawings. The area of the myocardium which is non-perfused as a result of the arterial occlusion is arrowed and may clearly be seen in the image.

## Claims

1. A method of first pass, contrast enhanced organ perfusion magnetic resonance imaging of a human or vascularized animal subject, wherein an MR contrast agent composition is administered into the vasculature of said subject and magnetic resonance images are generated of slices through a vascularized organ of said subject during the passage of said agent through the vasculature of said organ, and wherein said contrast agent comprises a particulate superparamagnetic metal oxide and said images are generated using pre-saturation slabs and a T₂-weighted Turbo spin-echo sequence.

2. A method of generating MR images of a human or vascularized animal subject, previously administered with an MR contrast agent composition comprising a particulate superparamagnetic metal oxide into the vasculature of said subject, wherein said method comprises generating a first pass contrast enhanced organ perfusion magnetic resonance image of slices through the vascularized organ of said subject, and wherein said images are generated using pre-saturation slabs and a T₂-weighted Turbo spin echo sequence.

3. A method as claimed in claims 1 or 2 **characterised in that** said organ is the myocardium, the kidneys, the brain or the liver.

4. A method as claimed in claim 3 **characterised in that** said organ is the myocardium.

5. A method according to any of the previous claims **characterised in that** said sequence is used in a single shot acquisition.

6. A method according to any of the previous claims **characterised in that** said contrast agent composition comprises an ultra small superparamagnetic iron oxide.

7. A method according to any of the previous claims **characterised in that** said contrast agent composition comprises an ultra small superparamagnetic iron oxide having on its surface an optionally modified carbohydrate or polysaccharide or a derivative thereof.

8. A method as claimed in any of the previous claims **characterised in that** the contrast agent composition is administered by bolus injection over a period of less than 3 minutes into the vasculature of said subject.

9. The use of a particulate superparamagnetic metal oxide for the manufacture of an MR contrast agent composition for use in a method of diagnosis involving first pass contrast enhanced perfusion magnetic resonance imaging of perfusion deficits in a vascularized organ using pre-saturation slabs and a T₂-weighted Turbo spin-echo sequence.

10. The use according to claim 9 wherein the contrast agent composition comprises an ultra small superparamagnetic iron oxide.

11. The use according to claim 10 wherein the contrast agent composition comprises an ultra small superparamagnetic iron oxide having on its surface an optionally modified carbohydrate or polysaccharide or a derivative thereof.

12. The use according to any of claims 9 to 11 wherein the organ is the myocardium, the kidneys, the brain or the liver.

## Patentansprüche

1. Verfahren zur First-Pass kontrastverstärkten Organ-Magnetresonanz-Perfusionsbildgebung eines menschlichen oder vaskularisierten tierischen Subjektes, wobei eine MR-Kontrastmittelzusammensetzung in die Blutgefäße des Subjektes verabreicht wird und Magnetresonanzbilder aus Schichten durch ein maskularisiertes Organ des Subjektes während des Durchgangs des Mittels durch die Blutgefäße des Organs erzeugt werden, und wobei das Kontrastmittel ein partikuläres superparamagnetisches Metalloxid umfasst und die Bilder unter Verwendung von Presaturationsschichten und einer T₂-gewichteten Turbo-Spin-Echo Sequenz erzeugt werden.

2. Verfahren zum Erzeugen von Magnetresonanzbildern eines menschlichen oder vaskularisierten tierischen Subjektes, dem zuvor eine MR-Kontrastmittelzusammensetzung, umfassend ein partikuläres, superparamagnetisches Metalloxid, in die Blutgefäße des Subjektes verabreicht wird, wobei die Methode die Erzeugung eines First-Pass kontrastverstärkten Organ-Magnetresonanz-Perfusionsbildes von Schichten durch das vaskularisierte Organ des Subjektes umfasst, und wobei die Bilder unter Verwendung von Presaturationsschichten und einer T₂-gewichteten Turbo-Spin-Echo Sequenz erzeugt werden.

3. Verfahren nach Anspruch 1 oder 2, **dadurch gekennzeichnet, dass** das Organ das Myokardium, die Nieren, das Gehirn oder die Leber ist.

4. Verfahren nach Anspruch 3, **dadurch gekennzeichnet, dass** das Organ das Myokardium ist.

5. Verfahren nach einem der vorhergehenden Ansprüche, **dadurch gekennzeichnet, dass** die Sequenz in einer Einzelpuls-Aufnahme verwendet wird.

6. Verfahren nach einem der vorhergehenden Ansprüche, **dadurch gekennzeichnet, dass** die Kontrastmittelzusammensetzung ein ultrafeines, superparamagnetisches Eisenoxid umfasst.

7. Verfahren nach einem der vorhergehenden Ansprüche, **dadurch gekennzeichnet, dass** die Kontrastmittelzusammensetzung ein ultrafeines, superparamagnetisches Eisenoxid umfasst, welches auf seiner Oberfläche ein wahlweise modifiziertes Kohlenhydrat oder Polysaccharid oder ein Derivat davon umfasst.

8. Verfahren nach einem der vorhergehenden Ansprüche, **dadurch gekennzeichnet, dass** die Kontrastmittelzusammensetzung durch Bolusinjektion über eine Dauer von weniger als 3 Minuten in die Blutgefäße des Subjektes verabreicht wird.

9. Verwendung eines partikulären superparamagnetischen Metalloxides zur Herstellung einer MR-Kontrastmittelzusammensetzung zur Verwendung in einem Verfahren zur Diagnose, beinhaltend First-Pass kontrastverstärkte Magnetresonanzbildgebung von Perfusionsdefiziten in einem vaskularisierten Organ unter Verwendung von Presaturationsschichten und einer T₂-gewichteten Turbo-Spin-Echo Sequenz.

10. Verwendung nach Anspruch 9, wobei die Kontrastmittelzusammensetzung ultrafeines superparamagnetisches Eisenoxid umfasst.

11. Verwendung nach Anspruch 10, wobei die Kontrastmittelzusammensetzung ein ultrafeines superparamagnetisches Eisenoxid umfasst, welches auf seiner Oberfläche ein wahlweise modifiziertes Kohlenhydrat oder Polysaccharid oder ein Derivat davon aufweist.

12. Verwendung nach einem der Ansprüche 9 bis 11, wobei das Organ das Myokardium, die Nieren, das Gehirn oder die Leber ist.

## Revendications

1. Procédé d'imagerie par résonance magnétique de la perfusion d'organe à contraste amélioré du premier passage d'un sujet humain ou animal vascularisé, dans lequel une composition d'agent de contraste de RM est administrée dans le système vasculaire dudit sujet et il est produit des images de résonance magnétique de tranches à travers l'organe vascularisé dudit sujet au cours du passage dudit agent à travers le système vasculaire dudit organe, et dans lequel ledit agent de contraste comprend un oxyde métallique super-paramagnétique particulaire et lesdites images sont produites en utilisant des bandes de pré-saturation et une séquence à écho de spin très rapide pondérée en T₂.

2. Procédé de production d'images de RM d'un sujet humain ou animal vascularisé, une composition d'agent de contraste de RM comprenant un oxyde métallique super-paramagnétique particulaire ayant été administrée au préalable dans le système vasculaire dudit sujet, dans lequel ledit procédé comprend la production d'image par résonance magnétique de la perfusion d'organe à contraste amélioré du premier passage de tranches à travers l'organe vascularisée dudit sujet, et dans lequel lesdites images sont produites en utilisant des bandes de pré-saturation et une séquence à écho de spin très rapide pondérée en T₂.

3. Procédé selon les revendications 1 ou 2, **caractérisé en ce que** ledit organe est le myocarde, les reins, le cerveau ou le foie.

4. Procédé selon la revendication 3, **caractérisé en ce que** ledit organe est le myocarde.

5. Procédé selon l'une quelconque des revendications précédentes, **caractérisé en ce que** ladite séquence est utilisée avec acquisition par impulsion unique.

6. Procédé selon l'une quelconque des revendications précédentes, **caractérisé en ce que** ladite composition d'agent de contraste comprend un oxyde de fer super-paramagnétique de très petite taille de particule.

7. Procédé selon l'une quelconque des revendications précédentes, **caractérisé en ce que** ladite composition d'agent de contraste comprend un oxyde de fer super-paramagnétique de très petite taille de particule comportant sur sa surface un hydrate de carbone ou un polysaccharide éventuellement modifiés ou un dérivé de ceux-ci.

8. Procédé selon l'une quelconque des revendications précédentes, **caractérisé en ce que** la composition d'agent de contraste est administrée par injection de dose sur une période inférieure à 3 minutes dans le système vasculaire dudit sujet.

9. Utilisation d'un oxyde métallique super-paramagnétique particulaire pour la fabrication d'une composition d'agent de contraste par RM en vue d'une utilisation dans un procédé de diagnostic impliquant l'imagerie par résonance magnétique de la perfusion à contraste amélioré du premier passage de déficits de perfusion dans un organe vascularisé en utilisant des bandes de pré-saturation et une séquence à écho de spin très rapide pondérée en T₂.

10. Utilisation selon la revendication 9, dans laquelle la composition d'agent de contraste comprend un oxyde fer super-paramagnétique de très petite taille de particules.

11. Utilisation selon la revendication 10, dans laquelle la composition d'agent de contraste comprend un oxyde de fer super-paramagnétique de très petite taille de particules comportant sur sa surface un hydrate de carbone ou un polysaccharide éventuellement modifiés ou un dérivé de ceux-ci.

12. Utilisation selon l'une quelconque des revendications 9 à 11, dans laquelle l'organe est le myocarde, les reins, le cerveau ou le foie.
